# EUROPÄISCHE PATENTSCHRIFT

(11) **EP 2 977 745 B1**
(45) Veröffentlichungstag und Bekanntmachung des Hinweises auf die Patenterteilung: **17.04.2019**
(21) Anmeldenummer: 14178049.4
(22) Anmeldetag: 22.07.2014
(51) Int. Cl.: A61F 13/02, A61F 13/00, G01N 19/04, G09B 23/30

(54) **Vorrichtung und Verfahren zur Untersuchung der Verklebungsneigung von Wundauflagen**
Device and method for examining the adhesion tendency of wound dressings
Dispositif et procédé d'évaluation de la tendance à l'adhérence des pansements

(43) Veröffentlichungstag der Anmeldung: 27.01.2016
(73) Patentinhaber: Paul Hartmann AG, 89522 Heidenheim (DE)
(72) Erfinder: Deibler, Dr., Martin, 89542 Herbrechtingen (DE)
(74) Vertreter: Eibl, Christian

(56) Entgegenhaltungen:
- EP-A1- 1 923 077
- DE-A1-102008 064 510
- DE-C1- 4 118 200
- FR-A1- 2 788 342
- "CHEMINSTRUMENTS ADHESION/RELEASE TESTER MODEL AR-1000 OPERATING INSTRUCTIONS EZ-LAB SYSTEM", ChemInstruments Equipment Manual, 30. September 2009 (2009-09-30), XP055156560, Gefunden im Internet: URL:http://cheminstruments.com/media/wysiw yg/AR-1000_Manual.pdf [gefunden am 2014-12-03]

## Beschreibung

Die Erfindung betrifft eine Vorrichtung zur Untersuchung der Verklebungsneigung eines Prüfkörpers mit einer künstlichen Wunde. Die Vorrichtung umfasst ein Substrat mit einer Vertiefung, welche eine künstliche Wundtasche bildet und in welche ein Verbindungsmedium und der Prüfkörper eingebracht werden können, wobei das Verbindungsmedium im Wesentlichen zur Verklebung des Prüfkörpers mit dem Substrat beitragen soll. Die Erfindung betrifft gleichfalls ein Verfahren zur Untersuchung der Verklebungsneigung eines Prüfkörpers mit einer künstlichen Wunde.

Das Verkleben von Wundauflagen mit der zu behandelnden Wundoberfläche stellt ein häufiges Problem bei der Wundbehandlung dar. Die Verklebung einer Wundauflage mit einer Wundoberfläche kann durch verschiedene Vorgänge ausgelöst werden. Dabei spielt die fibrinabhängige Koagulation der Wundflüssigkeit beziehungsweise des Wundexsudates eine wesentliche Rolle. Die Wundflüssigkeit fungiert dann vereinfacht ausgedrückt als Klebstoff und verbindet die Wundoberfläche mit der Oberfläche der Wundauflage. Ein weiterer, die Verklebung einer Wundauflage auslösender Vorgang kann die Neubildung von Granulationsgewebe sowie von Gefäßen an der Wundoberfläche sein, insbesondere dann, wenn das Granulationsgewebe und die Gefäße in die Wundauflagen einwachsen.

Verklebt eine Wundauflage mit der Wundoberfläche und wird dann von der Wundoberfläche entfernt, zum Beispiel im Rahmen eines Verbandswechsels, kann dabei neu gebildetes Granulationsgewebe beschädigt werden. Hierdurch wird der Heilungsverlauf der Wunde beeinträchtigt und der Wundverschluss verzögert. Darüber hinaus ist das Entfernen einer mit der Wunde verklebten Wundauflage für den Patienten zumeist schmerzhaft. Es besteht daher das Bedürfnis, Wundauflagen zu entwickeln, welche nicht mit der Wunde verkleben. Um nicht-verklebende Wundauflagen entwickeln zu können, sind Prüfverfahren erforderlich, mit denen die Verklebungsneigung der Wundauflagen möglichst objektiv vorhergesagt werden kann. Diese Prüfverfahren lassen sich in zwei Kategorien unterscheiden: Zum einen Prüfverfahren unter Einsatz von Versuchstieren (in-vivo-Prüfverfahren). Zum anderen Prüfverfahren, bei denen auf den Einsatz von Versuchstieren verzichtet wird und stattdessen mit einer künstlichen Wunde gearbeitet wird (in-vitro-Prüfverfahren). Die vorliegende Erfindung betrifft in-vitro-Prüfverfahren.

Da die an der Verklebung von Wundauflagen beteiligten Vorgänge sehr komplex sind und sich nur näherungsweise in einer künstlichen Wunde nachbilden lassen, sind in-vivo-Prüfverfahren aussagekräftiger als in-vitro-Prüfverfahren. Dennoch sind in-vitro-Prüfverfahren sehr nützlich, um bei der Entwicklung von nicht-verklebenden Wundauflagen eine Vorauswahl treffen zu können. Durch eine auf den Ergebnissen der in-vitro-Prüfungen basierende Vorauswahl kann die Anzahl der erforderlichen in-vivo-Prüfungen reduziert werden.

In-vitro-Verfahren zur Untersuchung der Verklebungsneigung einer Wundauflage sind im Stand der Technik bekannt, zum Beispiel aus der wissenschaftlichen Veröffentlichung "The Adhesion of Wound Dressings - an Experimental Study" von J. T. Scales und G. D. Winter (Wound Healing, Proceedings of a Symposium, 12.-13. November 1959, Seiten 54-61). Das in dieser Veröffentlichung beschriebene Verfahren sieht vor, die auf ihre Verklebungsneigung zu untersuchenden Proben mit einer 33 %-igen Serumlösung zu beaufschlagen und auf eine Platte aufzubringen. Dabei ist die Platte mit Serum beschichtet. Die Ansätze werden in einem Trockenschrank bei einer Temperatur von 37 °C und einer relativen Luftfeuchtigkeit von 66 % inkubiert, wobei das Serum in Abhängigkeit von der Dauer der Inkubation unterschiedlich stark eintrocknet. Zur Bestimmung der Verklebungsneigung werden die Proben nach unterschiedlichen Inkubationszeiten von den Platten abgezogen und die hierfür erforderliche Kraft mit einem Tensiometer ermittelt. Da Serumlösung definitionsgemäß kein Fibrinogen beinhaltet, lässt sich mit diesem Verfahren die fibrinabhängige Koagulation der Wundflüssigkeit nicht hinreichend simulieren.

Ein weiteres in-vitro-Verfahren zur Untersuchung der Verklebungsneigung einer Wundauflage ist in der DE4118200C1 offenbart. Das Verfahren zeichnet sich dadurch aus, dass zunächst eine Agar/Kollagen-Matrix oder eine Agarose/KollagenMatrix in einer Petrischale hergestellt wird. Auf die Matrix werden Humanplasma, eine Kalziumchlorid-Lösung und Gerinnungsreagenz aufgebracht. In einem folgenden Inkubationsschritt bildet sich ein Fibrinnetz aus. Auf das sich bildende Fibrinnetz werden die auf ihre Verklebungsneigung zu untersuchenden Proben aufgelegt. Es wird weiter inkubiert, bis sich das Fibrinnetz vollständig ausgebildet hat. Schließlich werden die Proben mit einer Pinzette manuell von der Matrix mit dem Fibrinnetz abgezogen. Zur Auswertung werden mikroskopische Bilder aufgenommen. Mit dem in der DE4118200C1 offenbarten Verfahren werden die Verklebungskräfte nur indirekt und nicht als exakter Messwert in Form einer Kraftangabe ermittelt. Zudem ist das manuelle Abziehen der Proben mit einer Pinzette schwer standardisierbar und kann je nach Durchführung zu unterschiedlichen Ergebnissen führen.

Die EP1923077A1 beschreibt eine Testmethode mit der beurteilt werden kann, wie gut sich eine Wundauflage von einer in-vitro Fibrinschicht lösen lässt. Als Substrat wird eine Glasplatte mit einer Schaumstoffumrandung verwendet. Innerhalb der Schaumstoffumrandung wird die Fibrinschicht ausgebildet. Zur Prüfung wird das Probenmaterial auf die Glasplatte mit der Fibrinschicht aufgebracht und mittels einer Abzugsvorrichtung unter Messung des Kraftverlaufs wieder entfernt. Die DE102008064510A1 offenbart eine Messapparatur für Unterdrucktherapiesysteme für die Wundbehandlung umfassend eine Wundtasche mit Fluiddurchlässen. Die Wundtasche kann ein eine offene Porosität aufweisendes Material wie eine Glasfritte beinhalten.

Der vorliegenden Erfindung liegt die Aufgabe zugrunde, eine verbesserte Vorrichtung und ein verbessertes Verfahren zur Untersuchung der Verklebungsneigung einer Wundauflage zur Verfügung zu stellen und die Nachteile im Stand der Technik zu überwinden. Insbesondere liegt der vorliegenden Erfindung die Aufgabe zugrunde, eine Vorrichtung und ein Verfahren zur Untersuchung der Verklebungsneigung einer Wundauflage zur Verfügung zu stellen, um die Verklebungsneigung der Wundauflage in einem in-vitro-System exakt quantifizieren zu können. Weiterhin sollen die Vorgänge, die in-vivo zur Verklebung der Wundauflage beitragen, in einem in-vitro-System möglichst vollständig nachgebildet werden. Dabei sollten vor allem mechanische Wechselwirkungen zwischen der Wundoberfläche und der Wundauflage besser nachgebildet werden.

Die Erfindung löst die genannten Aufgaben mit einer Vorrichtung mit den Merkmalen von Anspruch 1 und einem Verfahren mit den Merkmalen von Anspruch 12. Eine Vorrichtung mit den Merkmalen von Anspruch 1 ist besonders geeignet, um die Verklebungsneigung einer Wundauflage zu bestimmen. Indem der Prüfkörper, das heißt das als Wundauflage vorgesehene und zu untersuchende Material, in eine künstliche Wundtasche mit einer strukturierten Oberfläche eingebracht wird, können in-vivo auftretende, mechanische Wechselwirkungen zwischen der Wundoberfläche und der Wundauflage besser nachgebildet werden. Unter diesen mechanischen Wechselwirkungen ist beispielsweise eine formschlüssige Verbindung wie das Verhaken einer Granulationsgewebe ausbildenden Wundoberfläche mit einer darauf platzierten porösen Wundauflage zu verstehen. Da beim Abziehen des Prüfkörpers aus der künstlichen Wundtasche sowohl die aufgewendete Kraft als auch der zurückgelegte Weg gemessen werden, erlaubt die erfindungsgemäße Vorrichtung eine objektive und exakte Quantifizierung der Verklebungsneigung.

Die Erfindung betrifft demnach gemäß einem ersten Aspekt eine Vorrichtung zur Untersuchung der Verklebungsneigung eines Prüfkörpers mit einer künstlichen Wunde nach Anspruch 1.

Weiterhin betrifft die Erfindung gemäß einem zweiten Aspekt ein Verfahren zur Untersuchung der Verklebungsneigung eines Prüfkörpers mit einer künstlichen Wunde nach Anspruch 12.

Das Verfahren gemäß dem zweiten Aspekt der Erfindung kann mit der Vorrichtung gemäß dem ersten Aspekt der Erfindung durchgeführt werden.

Bei dem Prüfkörper handelt es sich um das Material beziehungsweise den Gegenstand, dessen Verklebungsneigung untersucht werden soll und dessen Verwendung als Wundauflage vorgesehen ist. Der Prüfkörper kann identisch mit einer gebrauchsfertigen Wundauflage, wie sie zur Anwendung am Patienten bereitgestellt wird, sein oder nur einen Teil einer solchen Wundauflage umfassen. Vorzugsweise umfasst der Prüfkörper einen Schaumstoff, ein Vlies, ein Gewebe, ein Gewirke, und/oder eine Folie. Mindestens eines dieser Materialien findet sich in nahezu jeder zurzeit kommerziell erhältlichen Wundauflage wieder. Die erfindungsgemäße Vorrichtung sowie das erfindungsgemäße Verfahren sind auch dazu geeignet, eine Wundauflage aus dem Bereich der Unterdrucktherapie auf ihre Verklebungsneigung zu untersuchen. Insbesondere trifft dies auf die in der Unterdrucktherapie als Druckverteiler eingesetzten offenzelligen Schaumstoffe zu, welche aufgrund ihrer porösen, gas-und flüssigkeitsdurchlässigen Struktur besonders häufig mechanische Wechselwirkungen mit der Wundoberfläche eingehen.

Die erfindungsgemäße Vorrichtung umfasst ein Substrat mit einer Vertiefung, welche eine künstliche Wundtasche bildet. In die Vertiefung kann ein Verbindungsmedium und der Prüfkörper eingebracht werden. Das Verbindungsmedium soll im Wesentlichen zur Verklebung des Prüfkörpers mit dem Substrat beitragen. Vorzugsweise werden erst das Verbindungsmedium und danach der Prüfkörper ohne zeitliche Verzögerung in die Vertiefung des Substrates eingebracht. Bei dem Substrat handelt es sich um einen Feststoff. Das Substrat kann beispielsweise ein Glas, ein Metall, einen keramischen Werkstoff, ein biologisches Polymer wie Zellulose und/oder einen Kunststoff umfassen. In das Substrat wird eine Vertiefung eingebracht, damit das zum Zeitpunkt des Einbringens in die Vertiefung in der Regel flüssige Verbindungsmedium nicht abfließen kann. Daher sollte die Vertiefung mindestens so groß sein, dass sie das Volumen des eingesetzten Verbindungsmediums sicher aufnehmen kann. Die Vertiefung muss jedoch nicht notwendigerweise so groß sein, um den Prüfkörper vollständig aufzunehmen. Damit ist gemeint, dass der Prüfkörper zwar in die Vertiefung eingebracht und dabei mit dem Verbindungsmedium sowie mit der Oberfläche der Vertiefung in Berührung gebracht wird, aber aus der Vertiefung noch herausragen kann. Das Substrat und die Vertiefung können verschiedene Formen besitzen. Üblicherweise besitzt das Substrat die Form eines flachen Quaders. Der durch die Vertiefung gebildete Raum ist ähnlich wie das Substrat üblicherweise im Wesentlichen quaderförmig. Das Substrat kann fester Bestandteil der erfindungsgemäßen Vorrichtung sein, wobei es dann mit dem Mittel zum Abziehen des Prüfkörpers von dem Substrat dauerhaft verbunden ist. Vorteilhaft ist jedoch, wenn das Substrat von den übrigen Komponenten der erfindungsgemäßen Vorrichtung zeitweise gelöst werden kann.

Wesentlich an der erfindungsgemäßen Vorrichtung ist, dass die Vertiefung in dem Substrat einen Bereich mit einer strukturierten Oberfläche aufweist. Unter einer strukturierten Oberfläche soll dabei im Wesentlichen eine Oberfläche verstanden werden, die nicht glatt, sondern rau ist. Die strukturierte Oberfläche weist also regelmäßig oder unregelmäßig angeordnete Unebenheiten auf. Die strukturierte Oberfläche ist in der Lage, mechanische Wechselwirkungen mit dem Verbindungsmedium und dem Prüfkörper einzugehen und trägt zu einer besseren Simulation der in-vivo stattfindenden Vorgänge, die zur Verklebung von Wundauflagen führen, bei. Insbesondere Granulationsgewebe, welches mit der Wundauflage mechanisch interagiert und sich so zum Beispiel mit der Wundauflage verhakt, kann durch die strukturierte Oberfläche besser nachgebildet werden. Vorzugsweise ist die Vertiefung im Wesentlichen vollständig mit der strukturierten Oberfläche ausgestattet. Damit ist sichergestellt, dass die Oberfläche des Prüfkörpers vollständig mit der strukturierten Oberfläche der Vertiefung in Berührung kommen kann. Es ist jedoch auch denkbar, nur einen Teilbereich der Oberfläche zu strukturieren, zum Beispiel um weniger stark granulierende Wunden zu simulieren. Ebenso ist denkbar, die Vertiefung in dem Substrat mit zwei oder mehreren Bereichen, die unterschiedlich strukturierte Oberflächen aufweisen, auszustatten.

In einer bevorzugten Ausführungsform der Erfindung wird die strukturierte Oberfläche durch eine in die Vertiefung des Substrates eingebrachte zusätzliche Materiallage gebildet. Dabei ist vorteilhaft, dass die strukturierte Oberfläche auch ein anderes Material als das Substrat umfassen kann. Die zusätzliche Materiallage muss in der Vertiefung fixiert werden, damit die Materiallage beim Abziehen des Prüfkörpers in der Vertiefung verbleibt. Die Materiallage kann ein Glas, ein Metall, einen keramischen Werkstoff, ein biologisches Polymer wie Zellulose und/oder einen Kunststoff umfassen. Es ist aber auch denkbar, dass die Materiallage ein tierisches oder menschliches Gewebe, insbesondere tierische oder menschliche Haut, umfasst.

Eine alternative, besonders bevorzugte Ausführungsform der Erfindung sieht vor, die strukturierte Oberfläche durch ein mechanisches Behandlungsverfahren in der Vertiefung des Substrates zu erzeugen. Ein geeignetes mechanisches Behandlungsverfahren ist das Sandstrahlen. Es können auch Behandlungsverfahren auf chemischer Basis wie Ätzen oder elektroerosive Behandlungsverfahren zum Einsatz kommen. Schließlich ist vorstellbar, das Substrat mitsamt der Vertiefung und der strukturierten Oberfläche durch einen Gießprozess herzustellen.

Die strukturierte Oberfläche weist erfindungsgemäß eine maximale Rautiefe von 10 µm bis 800 µm auf. Bevorzugt weist die strukturierte Oberfläche eine maximale Rautiefe von 10 µm bis 400 µm, noch mehr bevorzugt eine maximale Rautiefe von 10 µm bis 200 µm und ganz besonders bevorzugte eine maximale Rautiefe von 20 µm bis 150 µm auf. Insbesondere weist die strukturierte Oberfläche eine maximale Rautiefe von 20 µm bis 100 µm, von 30 µm bis 60 µm oder von 40 µm bis 50 µm auf. Die maximale Rautiefe wird nach DIN EN ISO 4287:1998 + AC:2008 + A1:2009 D und DIN EN ISO 4288:1997 D gemessen, wobei unter der maximalen Rautiefe der größte Rz-Wert der fünf Rz-Werte aus den fünf Einzelmessstrecken innerhalb der Messstrecke zu verstehen ist. Mit dem Rz-Wert ist dabei wie in der DIN EN ISO 4287:1998 + AC:2008 + A1:2009 D im Punkt 4.1.3. auf Seite 12 beschrieben die Summe aus der Höhe der größten Profilspitze und der Tiefe des größten Profiltales innerhalb einer Einzelmessstrecke gemeint. Für die maximale Rautiefe nach der zuvor genannten Definition wird im vorliegenden Dokument die Abkürzung Rz1max verwendet. An der strukturierten Oberfläche sollen fünf Messungen durchgeführt werden (abweichend von der in der DIN EN ISO 4288:1997 D im Punkt A.3.2 auf Seite 8 gemachten Angabe). Auf die ermittelten Rz1max-Werte ist die Höchstwert-Regel anzuwenden (DIN EN ISO 4288:1997 D, Seite 5, Punkt 5.3). Das heißt, keiner der in den Messungen ermittelten Rz1max-Werte darf den im vorliegenden Dokument beanspruchten oberen Rz1max-Wert überschreiten. Entsprechendes gilt für den beanspruchten unteren Rz1max-Wert. Es darf also auch keiner der in den Messungen ermittelten Rz1max-Werte den im vorliegenden Dokument beanspruchten unteren Rz1max-Wert unterschreiten.

Gemäß einer weiteren, bevorzugten Ausführungsform der Erfindung weist die strukturierte Oberfläche eine mittlere Rautiefe von 10 µm bis 800 µm, mehr bevorzugt eine mittlere Rautiefe von 10 µm bis 400 µm, noch mehr bevorzugt eine mittlere Rautiefe von 10 µm bis 200 µm und ganz besonders bevorzugte eine mittlere Rautiefe von 20 µm bis 150 µm auf. Insbesondere weist die strukturierte Oberfläche eine mittlere Rautiefe von 20 µm bis 100 µm, von 20 µm bis 50 µm oder von 30 µm bis 45 µm auf. Die mittlere Rautiefe wird gleichfalls nach DIN EN ISO 4287:1998 + AC:2008 + A1:2009 D und DIN EN ISO 4288:1997 D gemessen. Unter der mittleren Rautiefe ist der Mittelwert der fünf Rz-Werte aus den fünf Einzelmessstrecken innerhalb der Messstrecke zu verstehen. Mit dem Rz-Wert ist dabei wie in der DIN EN ISO 4287:1998 + AC:2008 + A1:2009 D im Punkt 4.1.3. auf Seite 12 beschrieben die Summe aus der Höhe der größten Profilspitze und der Tiefe des größten Profiltales innerhalb einer Einzelmessstrecke gemeint. Wie zuvor für die maximale Rautiefe beschrieben, sollen auch bei der Bestimmung der mittleren Rautiefe fünf Messungen durchgeführt werden, wobei die erhaltenen Werte für die mittlere Rautiefe den im vorliegenden Dokument beanspruchten Bereich gleichfalls weder über- noch unterschreiten dürfen.

Das mit dem Prüfkörper in die Vertiefung des Substrates eingebrachte Verbindungsmedium soll im Wesentlichen zur Verklebung des Prüfkörpers mit dem Substrat beitragen. Insbesondere soll das Verbindungsmedium den Einfluss der Wundflüssigkeit auf die Verklebung einer Wundauflage simulieren. Entsprechend soll das Verbindungsmedium ähnliche Eigenschaften wie die Wundflüssigkeit aufweisen. Das Verbindungsmedium ist vorzugsweise eine Flüssigkeit, deren Viskosität sich nach dem Einbringen in die Vertiefung des Substrates verändern kann, zum Beispiel durch einen Trocknungsprozess, einen Polymerisationsprozess oder einen Koagulationsprozess. Substanzen oder Substanzgemische, welche keinerlei Verklebungsreaktion zwischen zwei Materialoberflächen bewirken können, kommen als Verbindungsmedium für die erfindungsgemäße Vorrichtung und das erfindungsgemäße Verfahren nicht in Betracht.

In einer bevorzugten Ausführungsform der Erfindung umfasst das Verbindungsmedium eine tierische oder menschliche Körperflüssigkeit. Die tierische oder menschliche Körperflüssigkeit kann mit Kalzium-Lösung koaguliertes Blutplasma beziehungsweise mit Kalziumlösung zur Koagulation gebrachtes Blutplasma umfassen. Dabei wird die Koagulation vorzugsweise erst dann ausgelöst, wenn das Blutplasma in die Vertiefung des Substrates eingebracht wird. In einer weiteren bevorzugten Ausführungsform der Erfindung umfasst das Verbindungsmedium eine Flüssigkeit, welche in einer tierischen oder menschlichen Körperflüssigkeit vorkommende Bestandteile beinhaltet. Diese Ausführungsform berücksichtigt die Möglichkeit, auf den direkten Einsatz von Körperflüssigkeiten für das Verbindungsmedium zu verzichten und das Verbindungsmedium synthetisch herzustellen. Generell kann das Verbindungsmedium auch Mikroorganismen sowie tierischen oder menschliche Zellen, wie sie auch in der Wundflüssigkeit zu finden sind, umfassen.

Die erfindungsgemäße Vorrichtung umfasst ein Mittel zum Abziehen des Prüfkörpers von dem Substrat. Dieses Mittel gestattet, die bei dem Abziehen des Prüfkörpers von dem Substrat aufgewendete Kraft und den zurückgelegten Weg zu messen, wodurch die Verklebungsneigung exakt quantifiziert werden kann. Bei dem Mittel zum Abziehen des Prüfkörpers kann es sich um eine übliche Universalprüfmaschine handeln. Eine geeignete Universalprüfmaschine mit einem Biegebalken-Kraftaufnehmer zur Verwendung in der erfindungsgemäßen Vorrichtung wird beispielsweise von der Firma Zwick Roell (Ulm, Deutschland) unter der Bezeichnung ProLine Z010 angeboten.

Der Prüfkörper wird bevorzugt in einem Winkel von 0° bis 180°, mehr bevorzugt in einem Winkel von 20° bis 160°, noch mehr bevorzugt in einem Winkel von 45° bis 135° und ganz besonders bevorzugt in einem Winkel von 90° von dem Substrat abgezogen. Der Winkel bezieht sich dabei auf die Lage des Prüfkörpers in der Vertiefung des Substrates vor dem Abziehen, wobei der Prüfkörper weitestgehend flach und nicht etwa gebogen in der Vertiefung des Substrates zum Liegen kommen muss. Alle Winkelangaben hinsichtlich der Abzugsrichtung im vorliegenden Dokument sind auf diese Art und Weise zu verstehen.

Das Substrat kann in der erfindungsgemäßen Vorrichtung auch in einem beweglichen Aufnahmemittel lösbar positioniert werden. Um das Substrat an dem beweglichen Aufnahmemittel fixieren zu können, umfasst das Aufnahmemittel vorzugsweise mindestens einen Befestigungsmechanismus. Dieser Befestigungsmechanismus kann beispielsweise in der Art eines Spannmechanismus ausgebildet sein. Die Beweglichkeit des Aufnahmemittels lässt sich beispielsweise durch einen von dem Aufnahmemittel umfassten beweglichen Schlitten realisieren. Vorzugsweise bewegt sich das Aufnahmemittel bei dem Abziehen des Prüfkörpers von dem Substrat derart, dass die zum Abziehen des Prüfkörpers angewendete Kraft stets in einem im Wesentlichen gleichbleibenden Winkel, vorzugsweise in einem im Wesentlichen gleichbleibenden Winkel von 90°, zur Oberfläche des Prüfkörpers einwirkt. Solange das Mittel zum Abziehen des Prüfkörpers von dem Substrat seine Position konstant hält, ist es nur mit dem vorgenannten beweglichen Aufnahmemittel möglich, den Prüfkörper in einem gleichbleibenden Winkel von 90° zur Oberfläche des Prüfkörpers abzuziehen. In der zuletzt beschriebenen Ausführungsform wird die Bewegung des Aufnahmemittels mit einem steuerbaren Antriebsmittel unterstützt oder bewirkt. Dabei wird das Antriebsmittel mit dem Mittel zum Abziehen des Prüfkörpers von dem Substrat derart koordiniert, dass die Geschwindigkeit, mit der der Prüfkörper von dem Substrat abgezogen wird, mit der Geschwindigkeit, mit der das Antriebsmittel das Aufnahmemittel bewegt, übereinstimmt. Für den beschriebenen Zweck geeignete Antriebsmittel sind dem Fachmann bekannt, zum Beispiel Elektromotoren.

In einer bevorzugten Ausführungsform der Erfindung umfasst die Vorrichtung eine Klimakammer. Die Klimakammer dient zur Einstellung der Umweltbedingungen während der Untersuchung der Verklebungsneigung. Als Umweltbedingungen sind dabei insbesondere die Temperatur, die Luftfeuchtigkeit sowie die Kohlendioxid-Konzentration zu nennen. Durch die Verwendung der Klimakammer kann die Verklebungsreaktion zwischen Substrat und Prüfkörper beeinflusst und die Reproduzierbarkeit der Versuchsergebnisse verbessert werden.

Das erfindungsgemäße Verfahren kann den Schritt der Inkubation des Substrates mit dem Verbindungsmedium und dem Prüfkörper umfassen. Dieser Inkubationsschritt ist im Wesentlichen dann erforderlich, wenn das Verbindungsmedium Zeit für die Verklebungsreaktion benötigt. Vorzugsweise wird die Inkubation in einer Klimakammer durchgeführt. Insofern sich das Substrat aus der für das Verfahren verwendeten Vorrichtung lösen lässt, kann die Inkubation auch in einer separaten Klimakammer durchgeführt werden. Es kann insbesondere bei einem hydrophilen und saugenden Prüfkörper vorteilhaft sein, den Prüfkörper während der Inkubation mit einem Gewicht gleichmäßig zu beschweren. Damit kann der Kontakt des Prüfkörpers mit der strukturierten Oberfläche sichergestellt werden.

Das erfindungsgemäße Verfahren kann mit der erfindungsgemäßen Vorrichtung sowie deren bevorzugten Ausführungsformen durchgeführt werden. Alle zuvor gemachten Ausführungen hinsichtlich des Prüfkörpers, des Verbindungsmediums, des Substrates, der Vertiefung, der strukturierten Oberfläche und des Mittels zum Abziehen des Prüfkörpers sind auch für das erfindungsgemäße Verfahren zutreffend.

### Figuren

Nachstehend wird die erfindungsgemäße Vorrichtung anhand von Zeichnungen sowie Messdaten näher erläutert. Zudem werden in den Figuren Versuchsergebnisse, die unter Verwendung des erfindungsgemäßen Verfahrens erzielt wurden, beispielhaft dargestellt. Die Erfindung soll nicht auf die in den Zeichnungen oder in der Beschreibung der Zeichnungen dargestellten Ausgestaltungen reduziert verstanden werden. Vielmehr umfasst die Erfindung auch Kombinationen der Einzelmerkmale der alternativen Formen.
**Figur 1a** zeigt eine Wundauflage in einer Wunde.
**Figur 1b** zeigt einen Prüfkörper in einer bevorzugten Ausführungsform der erfindungsgemäßen Vorrichtung.
**Figur 2a** zeigt, wie die Wundauflage aus Figur 1a von der Wunde entfernt wird.
**Figur 2b** zeigt, wie der Prüfkörper aus Figur 1b von der künstlichen Wundtasche der Vorrichtung aus Figur 1b abgezogen wird.
**Figur 3** zeigt ein Weg-Kraft-Diagramm als Ergebnis eines Abzugsversuches.
**Figur 4** zeigt ein Weg-Kraft-Diagramm als Ergebnis von Abzugsversuchen mit drei unterschiedlichen Prüfkörpern.
**Figur 5** zeigt ein Diagramm, in dem die maximale Verklebungskraft eines Prüfkörpers in Abhängigkeit von dem Proteingehalt sowie von dem Feuchtigkeitsgehalt des Verbindungsmediums dargestellt ist.
**Figuren 6a bis 6g** zeigen Rauheitsprofile eines Substrates der erfindungsgemäßen Vorrichtung.

### Figurenlegende

- 1: Wundoberfläche
- 2: Wundauflage
- 3: Prüfkörper
- 4: Substrat
- 5: Vertiefung
- 6: Verbindungsmedium
- 7: Spannmechanismus des Aufnahmemittels
- 8: Schlitten des Aufnahmemittels
- 9: Basiselement des Schlittens
- 10: maximale Verklebungskraft

### Beschreibung der Figuren

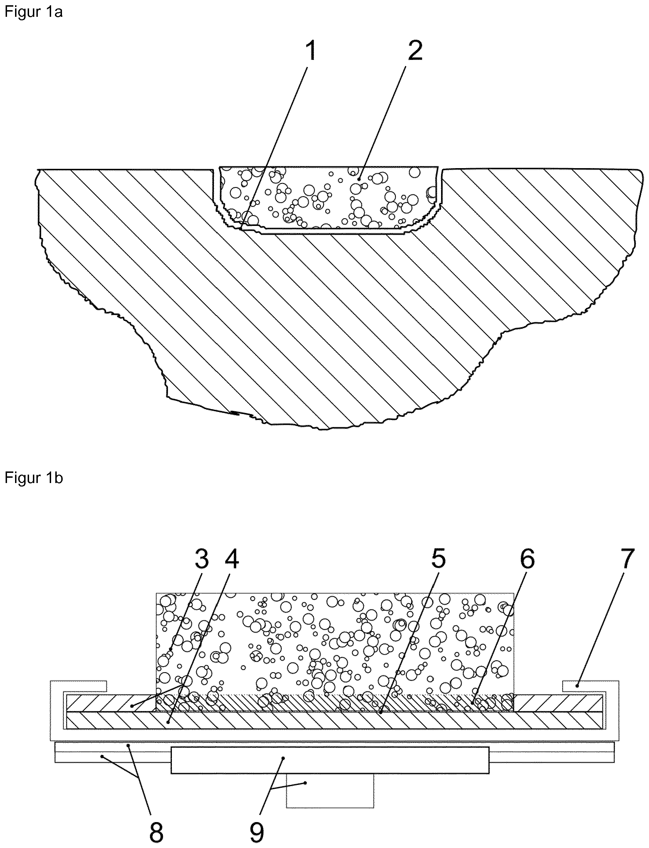

Figur 1a zeigt in schematischer Darstellung eine tiefe, offene Wunde in der Haut eines Menschen. Auf der Wundoberfläche (1) wird eine Wundauflage (2) positioniert, um die Wunde auszufüllen. Bei der Wundauflage (2) kann es sich beispielsweise um einen offenzelligen, retikulierten Polyurethanschaumstoff handeln. Durch von der Wunde abgegebene Wundflüssigkeit und neu gebildetes Granulationsgewebe kann die Wundauflage (2) mit der Wunde verkleben. Die in Figur 1a dargestellte Situation kommt häufig in der Unterdrucktherapie vor. Der Polyurethanschaumstoff wird dabei als Druckverteiler eingesetzt.

Figur 1b zeigt eine bevorzugte Ausführungsform der erfindungsgemäßen Vorrichtung, mit der die Verklebungsneigung der Wundauflage (2) in-vitro bestimmt werden kann. Zur Vereinfachung sind nicht alle Komponenten der Vorrichtung dargestellt. Beispielsweise ist das Mittel zum Abziehen des Prüfkörpers nicht dargestellt. Bei diesem Mittel kann es sich um die Universalprüfmaschine ProLine Z010 mit einem Biegebalken-Kraftaufnehmer (KAP-Z 10 N/100 N) der Firma Zwick Roell handeln. Der in der Vorrichtung positionierte Prüfkörper (3) besteht wie die Wundauflage (2) zum Beispiel aus einem offenzelligen, retikulierten Polyurethanschaumstoff und weist die Form eines Quaders auf. Die Vorrichtung umfasst ein flaches und quaderförmiges Substrat (4) mit einer Vertiefung (5), welche eine künstliche Wundtasche bildet und in welche ein Verbindungsmedium (6) und der Prüfkörper (3) eingebracht werden können. Der Untergrund der Vertiefung (5), auf dem der Prüfkörper (3) positioniert wird, ist rechteckig und der durch die Vertiefung (5) gebildete Raum gleichfalls quaderförmig. Ein solcher Grundkörper für das Substrat (4) kann erhalten werden, indem zwei Platten übereinander geklebt werden, wobei eine der Platten rahmenartig ausgeschnitten ist. Das Substrat (4) kann zum Beispiel aus Glas bestehen. Weiterhin kann das Substrat (4) eine Höhe von 10 mm, eine Auflagefläche mit einer Abmessung von 50 mm x 150 mm und eine 25 mm x 100 mm große Vertiefung, welche 5 mm in das Substrat hineinreicht, aufweisen. Die Vertiefung (5) weist einen Bereich mit einer strukturierten und somit angerauten Oberfläche auf (nicht aus der Figur ersichtlich). Der strukturierte Bereich kann sich über die gesamte Oberfläche der Vertiefung (5), also die Seitenwände sowie den Untergrund, oder nur einen Teil dieser Oberfläche erstrecken. Die strukturierte Oberfläche kann beispielsweise durch ein mechanisches Behandlungsverfahren wie Sandstrahlen mit Edelkorund F90 (Korngröße 0,125 mm bis 0,177 mm) als Strahlmittel in der Vertiefung (5) des Substrates (4) erzeugt werden. Bei dem Verbindungsmedium (6) kann es sich um bovines Citratplasma handeln, welches mit Kalziumchlorid-Lösung zur Koagulation gebracht wird. Das Volumen des Verbindungsmediums (6) sollte so bemessen sein, dass es vollständig von der Vertiefung (5) aufgenommen werden kann. Zudem sollte insbesondere im Falle eines hydrophilen, saugenden Prüfkörpers das Volumen des Verbindungsmediums so gewählt werden, dass nur ein Teil des Verbindungsmediums vom Prüfkörper absorbiert wird und noch genügend Verbindungsmedium in der Vertiefung für den Verklebungsprozess verbleibt. Das Volumen an einzusetzendem Verbindungsmedium ist bei der Untersuchung eines saugenden Prüfkörpers also in der Regel höher als bei der Untersuchung eines nicht-saugenden Prüfkörpers. Wie in Figur 1b angedeutet, ist der Prüfkörper (3) hinsichtlich seiner Länge und Breite exakt auf die Vertiefung (5) zugeschnitten. Außerdem ragt der Prüfkörper (3) aus der Vertiefung (5) heraus. Der zu untersuchende Prüfkörper kann auch kleiner dimensioniert werden, so dass der Prüfköper die Vertiefung nur teilweise ausfüllt. Dies ist insbesondere dann sinnvoll, wenn der Prüfkörper Verbindungsmedium absorbiert und sich dabei vergrößert. Die Vorrichtung umfasst weiterhin ein bewegliches Aufnahmemittel (7, 8), in welches das Substrat (4) lösbar positioniert wird. Um das Substrat (4) an dem Aufnahmemittel (7, 8) fixieren zu können, verfügt das Aufnahmemittel (7, 8) über einen Spannmechanismus (7). Die Beweglichkeit des Aufnahmemittels (7, 8) wird durch einen beweglichen Schlitten (8) realisiert, welcher von dem Basiselement (9) gehalten und geführt wird.

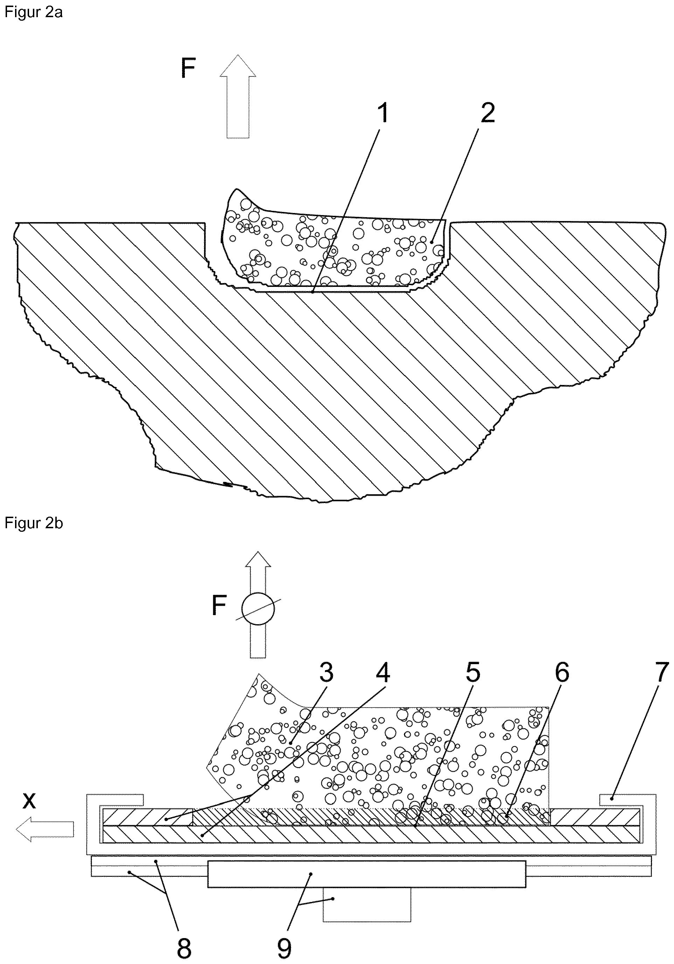

In Figur 2a ist dargestellt, wie die Wundauflage (2) aus Figur 1a von der Wunde entfernt wird. Dabei wird die Wundauflage (2) zum Beispiel mit einer Pinzette von einem behandelnden Arzt an einer Seite mit einer Kraft F angehoben und weggezogen. Figur 2b zeigt dann entsprechend, wie der Prüfkörper (3) aus Figur 1b von der künstlichen Wundtasche der Vorrichtung aus Figur 1b abgezogen wird, womit die Entfernung der Wundauflage (2) von der Wunde (Figur 2a) simuliert werden soll. Dabei bewegt sich das Aufnahmemittel (7, 8) derart in x-Richtung, dass die zum Abziehen des Prüfkörpers (3) angewendete Kraft F stets in einem Winkel von 90° zur Oberfläche des Prüfkörpers (3), bezogen auf die Lage des Prüfkörpers (3) in der Vertiefung (5) des Substrates (4) vor dem Abziehen, einwirkt. Das Mittel zum Abziehen des Prüfkörpers misst die bei dem Abziehen des Prüfkörpers (3) von dem Substrat (4) aufgewendete Kraft F und den zurückgelegten Weg x kontinuierlich.

### Ausführungsbeispiel 1

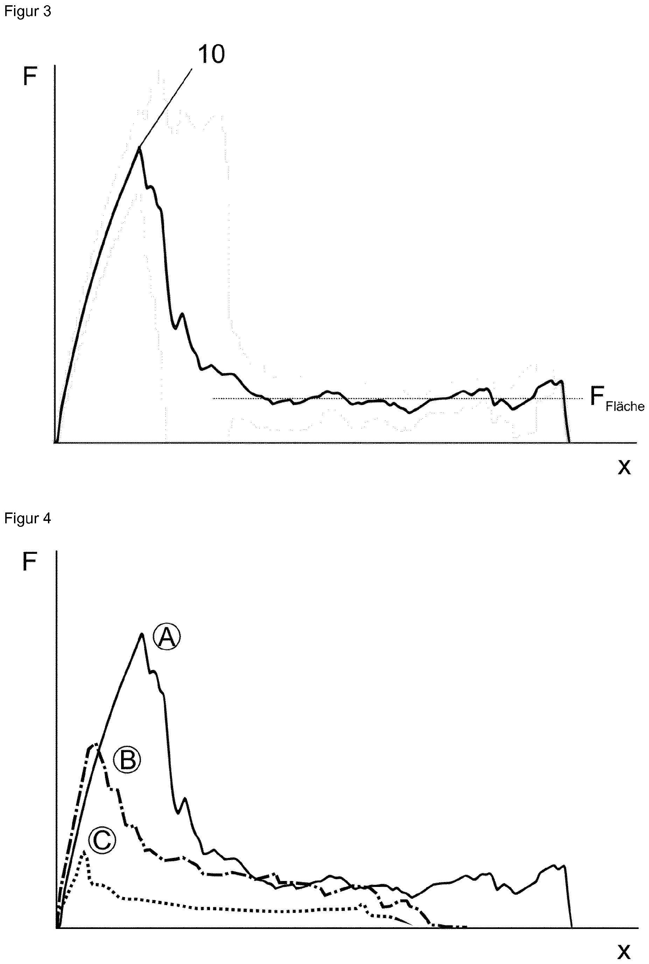

Figur 3 zeigt ein Weg-Kraft-Diagramm als Ergebnis eines Abzugsversuches. Der Versuch wird mit der zuvor beschriebenen Vorrichtung (Figuren 1b und 2b) und mit einer separaten Klimakammer durchgeführt. Der quaderförmige Prüfkörper besteht aus einem offenzelligen, retikulierten und hydrophoben Polyurethanschaumstoff. Das Substrat besteht aus Glas und besitzt die im Zusammenhang mit Figur 1b genannten Abmessungen (Substrat: 50 mm x 150 mm x 10 mm; Vertiefung: 25 mm x 100 mm x 5 mm). Die gesamte Oberfläche in der Vertiefung des Substrates wird durch Sandstrahlen mit Edelkorund F90 (Korngröße 0,125 mm bis 0,177 mm) strukturiert und somit angeraut. Der Prüfkörper ist entsprechend der Vertiefung gleichfalls 25 mm breit und 100 mm lang, ragt aber aus der Vertiefung wie in Figur 1b gezeigt heraus. Bei dem Mittel zum Abziehen des Prüfkörpers handelt es sich um die Universalprüfmaschine ProLine Z010 mit einem Biegebalken-Kraftaufnehmer (KAP-Z 10 N/100 N) der Firma Zwick Roell.

Im Folgenden wird der Versuchsablauf erläutert. Zunächst wird das koagulationsgehemmte, bovine Citratplasma mit einer 40 mM Kalziumchlorid-Lösung bei einer Temperatur von 34 °C in einem Verhältnis von 1:1 durch vorsichtiges Invertieren gemischt. Dadurch wird die Koagulation des bovinen Blutplasmas eingeleitet. Vier Milliliter der flüssigen Mischung werden als Verbindungsmedium in die Vertiefung des auf 34 °C vortemperierten Glassubstrates eingebracht. Unmittelbar danach wird der Prüfkörper aus Polyurethanschaumstoff gleichfalls in die Vertiefung der Glassubstrates eingebracht und manuell angedrückt, so dass sichergestellt ist, dass der Prüfkörper Kontakt zu der strukturierten Oberfläche der Vertiefung aufnimmt. Das Glassubstrat mit dem Verbindungsmedium und dem Prüfkörper wird dann für 20 Stunden bei einer Temperatur von 34 °C und einer relativen Luftfeuchtigkeit von 50 % in der Klimakammer inkubiert. Im Anschluss an die Inkubation wird das Glassubstrat mit dem Verbindungsmedium und dem Prüfkörper aus der Klimakammer entnommen und in dem Aufnahmemittel positioniert. Dabei wird das Glassubstrat mit dem Spannmechanismus innerhalb des Aufnahmemittels fixiert. Nach der Inkubation ist das Blutplasma koaguliert und trägt zur Verklebung des Prüfkörpers mit dem Substrat bei. Im nächsten Schritt wird eine Balkenklemme der Universalprüfmaschine an einem substratabgewandten Ende des Prüfkörpers befestigt. Schließlich wird der Prüfkörper von dem Substrat mit einer konstanten Geschwindigkeit von 100 mm/min abgezogen und die bei dem Abziehen des Prüfkörpers von dem Substrat aufgewendete Kraft F und der zurückgelegte Weg x kontinuierlich gemessen. Das Aufnahmemittel bewegt sich bei dem Abziehen des Prüfkörpers von dem Substrat derart, dass die zum Abziehen des Prüfkörpers angewendete Kraft F stets in einem Winkel von 90° zur Oberfläche des Prüfkörpers, bezogen auf die Lage des Prüfkörpers in der Vertiefung des Substrates vor dem Abziehen, einwirkt. Dieser Schritt läuft durch die schlittenartige Konstruktionsweise des Aufnahmemittels automatisch ab. Da der Prüfkörper eine Länge von 100 mm aufweist und mit einer Geschwindigkeit von 100 mm/min abgezogen wird, dauert die eigentliche Messung nur eine Minute. Die erhaltenen Messdaten werden in einem Weg-Kraft-Diagramm, wie es Figur 3 zeigt, ausgewertet. Das Diagramm enthält Informationen über die Verklebungsneigung der aus dem Polyurethanschaumstoff bestehenden Wundauflage, nämlich insbesondere die maximale Verklebungskraft und die Flächenverklebungskraft (F_{Fläche}). Die Flächenverklebungskraft ist die Kraft, der sich die Kurve aus Figur 3 annähert. Entsprechend handelt es sich bei der Flächenverklebungskraft um den interpolierten Grenzwert der Kurve aus Figur 3.

### Ausführungsbeispiel 2

Figur 4 zeigt ein Weg-Kraft-Diagramm als Ergebnis von Abzugsversuchen mit drei unterschiedlichen Prüfkörpern. Die Versuche werden wie im Ausführungsbeispiel 1 beschrieben durchgeführt. Prüfkörper A besteht aus einem offenzelligen, retikulierten, hydrophoben Polyurethanschaumstoff (wie in Ausführungsbeispiel 1). Bei Prüfkörper B handelt es sich um eine mit Polyvinylpyrrolidon chemisch modifizierten Variante des Prüfkörpers A. Prüfkörper C wird erhalten, indem Prüfkörper A mit einer Salbenmasse ausgestattet wird. Alle Prüfkörper besitzen dieselbe Form sowie dieselben Abmessungen. Wie aus dem Weg-Kraft-Diagramm der Figur 4 hervorgeht, wird sowohl für Prüfkörper B als auch für Prüfkörper C weniger Kraft zum Abziehen im Vergleich zu Prüfkörper A benötigt. Das bedeutet, dass Prüfkörper B und Prüfkörper C eine geringere Verklebungsneigung als Prüfkörper A besitzen, was mit in-vivo-Beobachtungen übereinstimmt.

### Ausführungsbeispiel 3

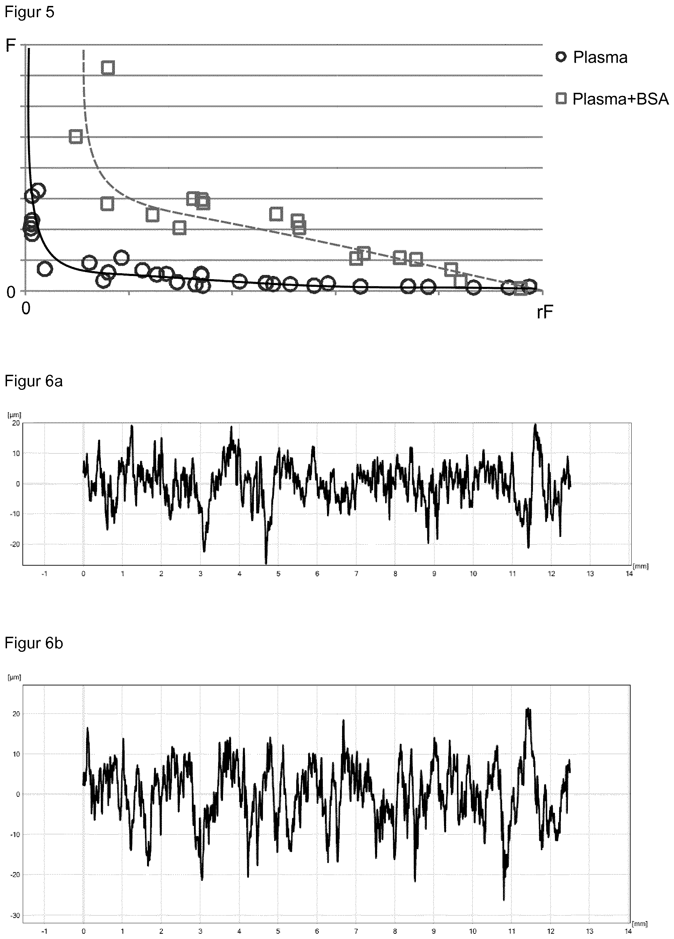

In weiteren Abzugsversuchen wird der Einfluss des Proteingehaltes des Verbindungsmediums sowie der Inkubationsbedingungen auf die Verklebungsneigung des Polyurethanschaumstoff-Prüfkörpers untersucht. Diese Versuche weichen von dem Versuch des Ausführungsbeispiels 1 in den nachfolgend genannten Punkten ab. Erstens wird als Verbindungsmedium auch eine Mischung aus einer BSA-Lösung und bovinem Citratplasma verwendet. Hierfür werden 1,7 g bovines Serumalbumin (BSA) in 5 ml 40 mM Kalziumchlorid-Lösung gelöst. Die erhaltene BSA-Lösung wird mit bovinem Citratplasma in einem Verhältnis von 1:1 durch vorsichtiges Invertieren gemischt, um das Verbindungsmedium zu erhalten. Zweitens wird die Inkubationszeit zwischen 1 und 20 Stunden variiert. Drittens wird das Glassubstrat mit dem Prüfkörper aus dem Polyurethanschaumstoff und mit dem Verbindungsmedium vor und auch nach der Inkubation gewogen, um den Feuchtigkeitsgehalt (rF) des Verbindungsmediums nach der Inkubation zu bestimmen. Die Untersuchungen haben ergeben, dass die maximale Verklebungskraft und somit die Verklebungsneigung des Prüfkörpers mit steigender Proteinkonzentration des Verbindungsmediums und sinkendem Wassergehalt des Verbindungsmediums zunimmt (Figur 5).

### Rauheitsmessungen

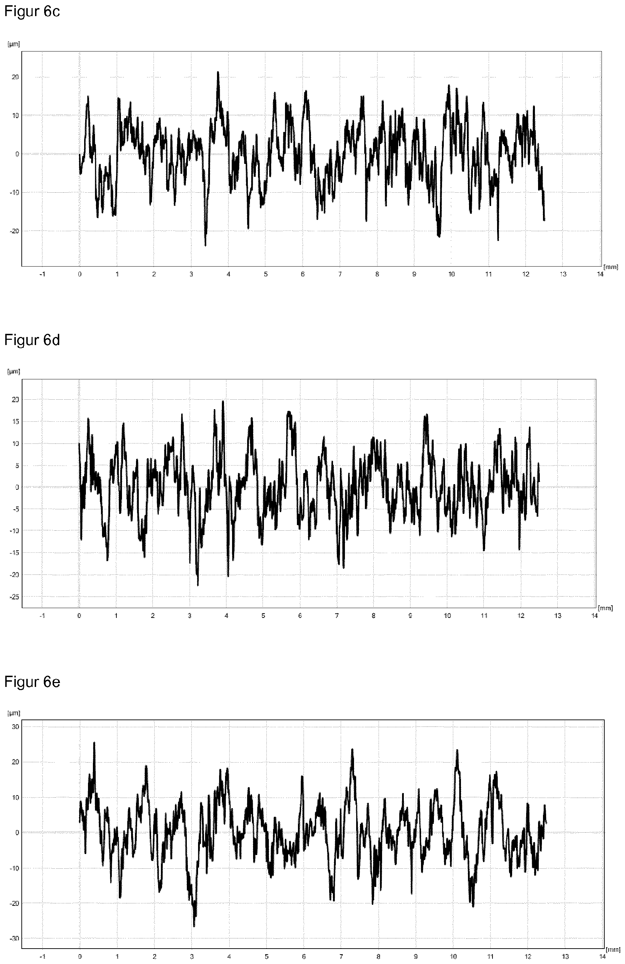

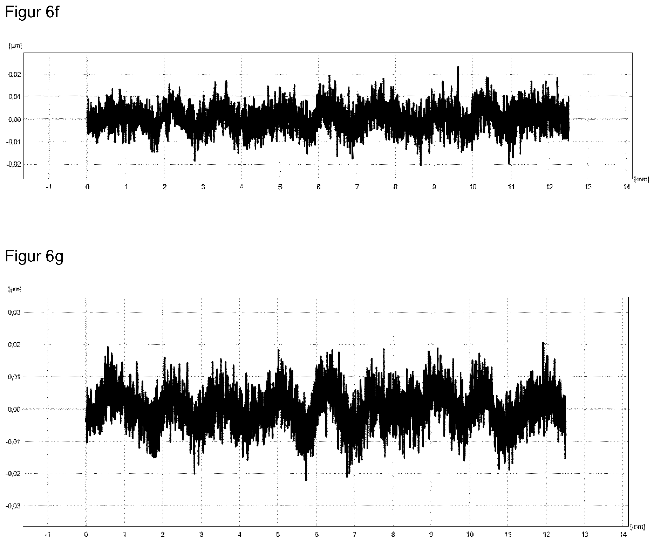

An einem Substrat der erfindungsgemäßen Vorrichtung werden Rauheitsmessungen durchgeführt. Das Substrat besteht aus Glas und weist die in Figur 1b gezeigte Form auf. Das Substrat wird erhalten, indem zwei Glasplatten mit den Abmessungen 67,5 mm x 185 mm x 5 mm mittels eines Epoxidharzklebers übereinander geklebt werden, wobei eine der Glasplatten rahmenartig ausgeschnitten ist. Das Innenmaß der rechteckig ausgeschnittenen Glasplatte und somit die Größe der Vertiefung beträgt 42,5 mm x 135 mm. Die gesamte Oberfläche in der Vertiefung des Substrates wird durch Sandstrahlen mit Edelkorund F90 (Korngröße 0,125 mm bis 0,177 mm) strukturiert und somit angeraut. Für die Rauheitsmessungen wird das Gerät SV-C 3000 der Firma Mitutoyo (Neuss, Deutschland) verwendet. Die Messungen erfolgen nach DIN EN ISO 4287:1998 + AC:2008 + A1:2009 D und DIN EN ISO 4288:1997 D. Die Grenzwellenlänge des Profilfilters λc beträgt 2,5 mm und die Grenzwellenlänge des Profilfilters λs beträgt 0,008 mm. Die Gesamtmessstrecke (ln) einer Messung beträgt 12,5 mm mit fünf Einzelmessstrecken (lrᵢ) von jeweils 2,5 mm Länge. Die Vorlaufstrecke und die Nachlaufstrecke einer Messung betragen jeweils 1,25 mm. Die durch Sandstrahlen angeraute Oberfläche in der Vertiefung des Substrates wird in fünf unterschiedlichen Bereichen vermessen (Figur 6a bis Figur 6e). Die glatte Oberfläche des Substrates außerhalb der Vertiefung wird in zwei unterschiedlichen Bereichen vermessen (Figur 6f und Figur 6g). Auf Basis der Messdaten werden Ra, Rz, Rz1max sowie RSm als Kenngrößen der Oberflächenbeschaffenheit des Substrates ermittelt. Die Messunsicherheit für Ra, Rz und Rz1max beträgt jeweils 10 %. Die Messunsicherheit für RSm beträgt 0,01 mm. Die nachfolgende Tabelle fasst die ermittelten Kenngrößen zusammen.

| | Arithmetischer Mittenrauwert (Ra)¹ | Mittlere Rautiefe (Rz)² | Maximale Rautiefe (Rz1max)³ | Mittlere Rillenbreite (RSm)⁴ |
|---|---|---|---|---|
| Messung 1, raue Oberfläche (Figur 6a) | 5,2704 µm | 35,0959 µm | 45,4560 µm | 0,34323 mm |
| Messung 2, raue Oberfläche (Figur 6b) | 5,9670 µm | 37,7496 µm | 47,7135 µm | 0,61169 mm |
| Messung 3, raue Oberfläche (Figur 6c) | 6,0456 µm | 37,8572 µm | 45,2433 µm | 0,48116 mm |
| Messung 4, raue Oberfläche (Figur 6d) | 5,5110 µm | 33,2553 µm | 42,1332 µm | 0,37097 mm |
| Messung 5, raue Oberfläche (Figur 6e) | 6,4958 µm | 41,8596 µm | 44,8969 µm | 0,45464 mm |
| Messung 6, glatte Oberfläche (Figur 6f) | 0,0043 µm | 0,0370 µm | 0,0439 µm | 0,05403 mm |
| Messung 7, glatte Oberfläche (Figur 6g) | 0,0047 µm | 0,0370 µm | 0,0405 µm | 0,05836 mm |

| | | | | |
|---|---|---|---|---|
| ¹Arithmetischer Mittenrauwert (Ra): arithmetischer Mittelwert der Beträge aller Profilwerte ²Mittlere Rautiefe (Rz): Mittelwert der fünf Rzᵢ-Werte aus den fünf Einzelmessstrecken lrᵢ innerhalb der Messstrecke In ³Maximale Rautiefe (Rz1max): Größter der fünf Rzᵢ-Werte aus den fünf Einzelmessstrecken lrᵢ innerhalb der Messstrecke In ⁴Mittlere Rillenbreite (RSm): Mittelwert der Breite der Profilelemente Xsᵢ | | | | |

Die Rauheitsmessungen ergeben, dass die durch Sandstrahlen angeraute Oberfläche in der Vertiefung des Substrates einen arithmetischen Mittenrauwert (Ra) in einem Bereich von 5,27 µm bis 6,50 µm, eine mittlere Rautiefe (Rz) in einem Bereich von 33,26 µm bis 41,86 µm, eine maximale Rautiefe (Rz1max) in einem Bereich von 42,13 µm bis 47,71 µm und eine mittlere Rillenbreite (RSm) in einem Bereich von 0,34 mm bis 0,61 mm aufweist. Alle ermittelten Kenngrößen streuen vergleichsweise gering. Die Rauheitsmessungen zeigen somit auch, dass das Sandstrahlen die Oberfläche der Vertiefung in dem Substrat gleichmäßig strukturiert und angeraut hat. Die Vergleichsmessungen 6 und 7 an der glatten Oberfläche des Substrates ergeben wie erwartet keine nennenswerte Rauheit, was insbesondere an den niedrigen Rz- und Rz1max-Werten zu erkennen ist.

## Patentansprüche

1. Vorrichtung zur Untersuchung der Verklebungsneigung eines Prüfkörpers (3) mit einer künstlichen Wunde, umfassend
- ein Substrat (4),
- ein Mittel zum Abziehen des Prüfkörpers (3) von dem Substrat (4), wobei die bei dem Abziehen des Prüfkörpers (3) von dem Substrat (4) aufgewendete Kraft und der zurückgelegte Weg messbar sind,
**dadurch gekennzeichnet, dass**
- das Substrat (4) eine Vertiefung (5) umfasst welche eine künstliche Wundtasche bildet und in welche ein Verbindungsmedium (6) und der Prüfkörper (3) eingebracht werden können, wobei das Verbindungsmedium (6) im Wesentlichen zur Verklebung des Prüfkörpers (3) mit dem Substrat (4) beitragen soll,
- die Vertiefung (5) einen Bereich mit einer strukturierten Oberfläche aufweist und wobei die strukturierte Oberfläche eine maximale Rautiefe von 10 µm bis 800 µm, gemessen nach DIN EN ISO 4287:1998 + AC:2008 + A1:2009 D und DIN EN ISO 4288:1997 D, aufweist.

2. Vorrichtung nach Anspruch 1, **dadurch gekennzeichnet, dass** das Substrat (4) ein Glas, ein Metall, einen keramischen Werkstoff, ein biologisches Polymer und/oder einen Kunststoff umfasst.

3. Vorrichtung nach einem der vorangehenden Ansprüche, **dadurch gekennzeichnet, dass** die strukturierte Oberfläche durch eine in die Vertiefung (5) eingebrachte Materiallage gebildet wird.

4. Vorrichtung nach Anspruch 1 oder 2, **dadurch gekennzeichnet, dass** die strukturierte Oberfläche durch ein mechanisches Behandlungsverfahren, vorzugsweise durch Sandstrahlen, in der Vertiefung (5) des Substrates (4) erzeugt wird.

5. Vorrichtung nach einem der vorangehenden Ansprüche, **dadurch gekennzeichnet, dass** die strukturierte Oberfläche eine maximale Rautiefe von 10 µm bis 200 µm, mehr bevorzugt 30 µm bis 60 µm oder noch mehr bevorzugt 40 µm bis 50 µm, gemessen nach DIN EN ISO 4287:1998 + AC:2008 + A1:2009 D und DIN EN ISO 4288:1997 D, aufweist.

6. Vorrichtung nach einem der vorangehenden Ansprüche, **dadurch gekennzeichnet, dass** die strukturierte Oberfläche eine mittlere Rautiefe von 10 µm bis 800 µm, vorzugsweise 10 µm bis 200 µm, mehr bevorzugt 20 µm bis 50 µm oder noch mehr bevorzugt 30 µm bis 45 µm, gemessen nach DIN EN ISO 4287:1998 + AC:2008 + A1:2009 D und DIN EN ISO 4288:1997 D, aufweist.

7. Vorrichtung nach einem der vorangehenden Ansprüche, **dadurch gekennzeichnet, dass** die Vorrichtung ein bewegliches Aufnahmemittel umfasst, wobei das Substrat (4) in dem beweglichen Aufnahmemittel lösbar positioniert werden kann.

8. Vorrichtung nach Anspruch 7, **dadurch gekennzeichnet, dass** das bewegliche Aufnahmemittel mindestens einen Befestigungsmechanismus, vorzugsweise einen Spannmechanismus (7), umfasst, so dass das Substrat (4) an dem Aufnahmemittel fixiert werden kann.

9. Vorrichtung nach Anspruch 7 oder 8, **dadurch gekennzeichnet, dass** das bewegliche Aufnahmemittel einen beweglichen Schlitten (8) umfasst.

10. Vorrichtung nach einem der Ansprüche 7 bis 9, **dadurch gekennzeichnet,**
- **dass** sich das Aufnahmemittel bei dem Abziehen des Prüfkörpers (3) von dem Substrat (4) derart bewegen kann, dass die zum Abziehen des Prüfkörpers (3) angewendete Kraft stets in einem im Wesentlichen gleichbleibenden Winkel, vorzugsweise in einem im Wesentlichen gleichbleibenden Winkel von 90°, zur Oberfläche des Prüfkörpers (3), bezogen auf die Lage des Prüfkörpers (3) in der Vertiefung (5) des Substrates (4) vor dem Abziehen, einwirken kann, und
- **dass** die Vorrichtung ein steuerbares Antriebsmittel umfasst,
- wobei die Bewegung des Aufnahmemittels mit dem steuerbaren Antriebsmittel unterstützt oder bewirkt werden kann, und
- wobei das Antriebsmittel mit dem Mittel zum Abziehen des Prüfkörpers (3) von dem Substrat (4) derart koordiniert werden kann, dass die Geschwindigkeit, mit der der Prüfkörper (3) von dem Substrat (4) abgezogen werden kann, mit der Geschwindigkeit, mit der das Antriebsmittel das Aufnahmemittel bewegen kann, übereinstimmt.

11. Vorrichtung nach einem der vorangehenden Ansprüche, **dadurch gekennzeichnet, dass** die Vorrichtung eine Klimakammer umfasst.

12. Verfahren zur Untersuchung der Verklebungsneigung eines Prüfkörpers (3) mit einer künstlichen Wunde, umfassend die Schritte
- Einbringen eines Verbindungsmediums (6) in eine Vertiefung (5) eines Substrates (4), wobei die Vertiefung (5) eine künstliche Wundtasche bildet und die Vertiefung (5) einen Bereich mit einer strukturierten Oberfläche aufweist und wobei das Verbindungsmedium (6) im Wesentlichen zur Verklebung des Prüfkörpers (3) mit dem Substrat (4) beitragen soll,
- Einbringen des Prüfkörpers (3) in die Vertiefung (5) des Substrates (4),
- optional eine Inkubation des Substrates (4) mit dem Verbindungsmedium (6) und dem Prüfkörper (3),
- Abziehen des Prüfkörpers (3) von dem Substrat (4), wobei die bei dem Abziehen des Prüfkörpers (3) von dem Substrat (4) aufgewendete Kraft und der zurückgelegte Weg gemessen werden,
wobei die strukturierte Oberfläche eine maximale Rautiefe von 10 µm bis 800 µm, gemessen nach DIN EN ISO 4287:1998 + AC:2008 + A1:2009 D und DIN EN ISO 4288:1997 D, aufweist.

13. Verfahren nach Anspruch 12, wobei das Verfahren mit einer Vorrichtung nach einem der Ansprüche 1 bis 11 durchgeführt wird.

14. Verfahren nach Anspruch 12 oder 13, wobei der Prüfkörper (3) eine zur Unterdrucktherapie von Wunden als Wundauflage vorgesehene Materiallage, insbesondere eine Materiallage aus einem offenzelligen Schaumstoff, umfasst.

15. Verfahren nach einem der Ansprüche 12 bis 14, wobei der Prüfkörper (3) einen Schaumstoff, ein Vlies, ein Gewebe, ein Gewirke, und/oder eine Folie umfasst.

16. Verfahren nach einem der Ansprüche 12 bis 15, wobei das Verbindungsmedium (6) eine tierische oder menschliche Körperflüssigkeit umfasst.

17. Verfahren nach Anspruch 16, wobei das Verbindungsmedium (6) mit Kalzium-Lösung koaguliertes Blutplasma umfasst.

18. Verfahren nach einem der Ansprüche 12 bis 15, wobei das Verbindungsmedium (6) eine Flüssigkeit, welche in einer tierischen oder menschlichen Körperflüssigkeit vorkommende Bestandteile beinhaltet, umfasst.

19. Verfahren nach einem der Ansprüche 12 bis 18, wobei der Prüfkörper (3) in einem Winkel von 0° bis 180°, bezogen auf die Lage des Prüfkörpers (3) in der Vertiefung (5) des Substrates (4) vor dem Abziehen, vom Substrat (4) abgezogen wird.

## Claims

1. Device for investigating the adhesion tendency of a test element (3) to an artificial wound, comprising
- a substrate (4),
- a means for removing the test element (3) from the substrate (4), where the distance travelled and the force expended on removal of the test element (3) from the substrate (4) can be measured,
**characterized in that**
- the substrate (4) comprises an indentation (5) which forms an artificial wound pocket and into which a connecting medium (6) and the test element (3) can be introduced, the connecting medium (6) being intended essentially to contribute to adhering the test element (3) to the substrate (4),
- the indentation (5) comprises a region having a structured surface and
where the structured surface has a maximum roughness depth of 10 µm to 800 µm, measured according to DIN EN ISO 4287:1998 + AC:2008 + A1:2009 D and DIN EN ISO 4288:1997 D.

2. Device according to Claim 1, **characterized in that** the substrate (4) comprises a glass, a metal, a ceramic material, a biological polymer and/or a plastic.

3. Device according to either of the preceding claims, **characterized in that** the structured surface is formed by a ply of material introduced into the indentation (5).

4. Device according to Claim 1 or 2, **characterized in that** the structured surface is generated by a mechanical treatment method, preferably by sand blasting, in the indentation (5) of the substrate (4) .

5. Device according to any of the preceding claims, **characterized in that** the structured surface has a maximum roughness depth of 10 µm to 200 µm, more preferably 30 µm to 60 µm or even more preferably 40 µm to 50 µm, measured according to DIN EN ISO 4287:1998 + AC:2008 + A1:2009 D and DIN EN ISO 4288:1997 D.

6. Device according to any of the preceding claims, **characterized in that** the structured surface has a mean roughness depth of 10 µm to 800 µm, preferably 10 µm to 200 µm, more preferably 20 µm to 50 µm or even more preferably 30 µm to 45 µm, measured according to DIN EN ISO 4287:1998 + AC:2008 + A1:2009 D and DIN EN ISO 4288:1997 D.

7. Device according to any of the preceding claims, **characterized in that** the device comprises a movable receiving means, where the substrate (4) can be positioned releasably in the movable receiving means.

8. Device according to Claim 7, **characterized in that** the movable receiving means comprises at least one fastening mechanism, preferably a tensioning mechanism (7), with the result that the substrate (4) can be fixed on the receiving means.

9. Device according to Claim 7 or 8, **characterized in that** the movable receiving means comprises a movable carriage (8).

10. Device according to any of Claims 7 to 9, **characterized**
- **in that** the receiving means, on removal of the test element (3) from the substrate (4), is able to move in such a way that the force expended for removing the test element (3) is able to act always at a substantially constant angle, preferably at a substantially constant angle of 90°, with respect to the surface of the test element (3), based on the position of the test element (3) in the indentation (5) of the substrate (4) before the removal, and
- **in that** the device comprises a controllable drive means,
- where the movement of the receiving means can be assisted or effected with the controllable drive means, and
- where the drive means can be coordinated with the means for removing the test element (3) from the substrate (4) in such a way that the rate at which the test element (3) can be removed from the substrate (4) coincides with the rate at which the drive means is able to move the receiving means.

11. Device according to any of the preceding claims, **characterized in that** the device comprises an environmental chamber.

12. Method for investigating the adhesion tendency of a test element (3) to an artificial wound, comprising the steps of
- introducing a connecting medium (6) into an indentation (5) of a substrate (4), where the indentation (5) forms an artificial wound pocket and the indentation (5) comprises a region having a structured surface, and where the connecting medium (6) is intended essentially to contribute to adhering the test element (3) to the substrate (4),
- introducing the test element (3) into the indentation (5) of the substrate (4),
- optionally incubating the substrate (4) with the connecting medium (6) and the test element (3),
- removing the test element (3) from the substrate (4), where the distance travelled and the force expended on removal of the test element (3) from the substrate (4) are measured,
where the structured surface has a maximum roughness depth of 10 µm to 800 µm, measured according to DIN EN ISO 4287:1998 + AC:2008 + A1:2009 D and DIN EN ISO 4288:1997 D.

13. Method according to Claim 12, where the method is carried out with a device according to any of Claims 1 to 11.

14. Method according to Claim 12 or 13, where the test element (3) comprises a ply of material intended as a wound dressing for the negative-pressure therapy of wounds, more particularly a ply of material comprising an open-celled foam.

15. Method according to any of Claims 12 to 14, where the test element (3) comprises a foam, a nonwoven, a woven or knitted fabric and/or a foil.

16. Method according to any of Claims 12 to 15, where the connecting medium (6) comprises an animal or human body fluid.

17. Method according to Claim 16, where the connecting medium (6) comprises blood plasma coagulated with calcium solution.

18. Method according to any of Claims 12 to 15, where the connecting medium (6) comprises a fluid which contains constituents occurring in an animal or human body fluid.

19. Method according to any of Claims 12 to 18, where the test element (3) is removed from the substrate (4) at an angle of 0° to 180°, based on the position of the test element (3) in the indentation (5) of the substrate (4) before the removal.

## Revendications

1. Dispositif pour évaluer la tendance à l'adhésion d'une éprouvette (3) avec une plaie artificielle, comprenant :
- un substrat (4),
- un moyen de pelage de l'éprouvette (3) du substrat (4), la force appliquée lors du pelage de l'éprouvette (3) du substrat (4) et la distance parcourue étant mesurables,
**caractérisé en ce que**
- le substrat (4) comprend un creux (5) qui forme une cavité de plaie artificielle et dans lequel un milieu de liaison (6) et l'éprouvette (3) peuvent être introduits, le milieu de liaison (6) devant essentiellement contribuer au collage de l'éprouvette (3) avec le substrat (4),
- le creux (5) comprend une zone ayant une surface structurée, la surface structurée présentant une profondeur de rugosité maximale de 10 µm à 800 µm, mesurée selon DIN EN ISO 4287:1998 + AC:2008 + A1:2009 D et DIN EN ISO 4288:1997 D.

2. Dispositif selon la revendication 1, **caractérisé en ce que** le substrat (4) comprend un verre, un métal, un matériau céramique, un polymère biologique et/ou une matière plastique.

3. Dispositif selon l'une quelconque des revendications précédentes, **caractérisé en ce que** la surface structurée est formée par une couche de matériau introduite dans le creux (5).

4. Dispositif selon la revendication 1 ou 2, **caractérisé en ce que** la surface structurée est formée par un procédé de traitement mécanique, de préférence par sablage, dans le creux (5) du substrat (4).

5. Dispositif selon l'une quelconque des revendications précédentes, **caractérisé en ce que** la surface structurée présente une profondeur de rugosité maximale de 10 µm à 200 µm, de manière davantage préférée de 30 µm à 60 µm ou de manière encore davantage préférée de 40 µm à 50 µm, mesurée selon DIN EN ISO 287:1998 + AC:2008 + A1:2009 D et DIN EN ISO 4288:1997 D.

6. Dispositif selon l'une quelconque des revendications précédentes, **caractérisé en ce que** la surface structurée présente une profondeur de rugosité moyenne de 10 µm à 800 µm, de préférence de 10 µm à 200 µm, de manière davantage préférée de 20 µm à 50 µm ou de manière encore davantage préférée de 30 µm à 45 µm, mesurée selon DIN EN ISO 4287:1998 + AC:2008 + A1:2009 D et DIN EN ISO 4288:1997 D.

7. Dispositif selon l'une quelconque des revendications précédentes, **caractérisé en ce que** le dispositif comprend un moyen de réception mobile, le substrat (4) pouvant être positionné de manière amovible dans le moyen de réception mobile.

8. Dispositif selon la revendication 7, **caractérisé en ce que** le moyen de réception mobile comprend au moins un mécanisme de fixation, de préférence un mécanisme de préhension (7), de telle sorte que le substrat (4) puisse être fixé au moyen de réception.

9. Dispositif selon la revendication 7 ou 8, **caractérisé en ce que** le moyen de réception mobile comprend un chariot mobile (8).

10. Dispositif selon l'une quelconque des revendications 7 à 9, **caractérisé en ce que**
- le moyen de réception peut être déplacé lors du pelage de l'éprouvette (3) du substrat (4), de telle sorte que la force appliquée pour le pelage de l'éprouvette (3) puisse toujours agir à un angle essentiellement constant, de préférence à un angle essentiellement constant de 90°, par rapport à la surface de l'éprouvette (3), par rapport à la position de l'éprouvette (3) dans le creux (5) du substrat (4) avant le pelage, et
- le dispositif comprend un moyen d'entraînement réglable,
- le mouvement du moyen de réception pouvant être supporté ou effectué avec le moyen d'entraînement réglable, et
- le moyen d'entraînement pouvant être coordonné avec le moyen de pelage de l'éprouvette (3) du substrat (4), de telle sorte que la vitesse à laquelle l'éprouvette (3) peut être pelée du substrat (4) corresponde à la vitesse à laquelle le moyen d'entraînement peut déplacer le moyen de réception.

11. Dispositif selon l'une quelconque des revendications précédentes, **caractérisé en ce que** le dispositif comprend une chambre climatique.

12. Procédé d'évaluation de la tendance à l'adhésion d'une éprouvette (3) avec une plaie artificielle, comprenant les étapes suivantes :
- l'introduction d'un milieu de liaison (6) dans un creux (5) d'un substrat (4), le creux (5) formant une cavité de plaie artificielle, et le creux (5) comprenant une zone ayant une surface structurée, et le milieu de liaison (6) devant essentiellement contribuer au collage de l'éprouvette (3) avec le substrat (4),
- l'introduction de l'éprouvette (3) dans le creux (5) du substrat (4),
- éventuellement une incubation du substrat (4) avec le milieu de liaison (6) et l'éprouvette (3),
- le pelage de l'éprouvette (3) du substrat (4), la force appliquée lors du pelage de l'éprouvette (3) du substrat (4) et la distance parcourue étant mesurées,
la surface structurée présentant une profondeur de rugosité maximale de 10 µm à 800 µm, mesurée selon DIN EN ISO 4287:1998 + AC:2008 + A1:2009 D et DIN EN ISO 4288:1997 D.

13. Procédé selon la revendication 12, dans lequel le procédé est réalisé avec un dispositif selon l'une quelconque des revendications 1 à 11.

14. Procédé selon la revendication 12 ou 13, dans lequel l'éprouvette (3) comprend une couche de matériau prévue en tant que compresse pour la peau pour le traitement par pression négative de plaies, notamment une couche de matériau en une mousse à cellules ouvertes.

15. Procédé selon l'une quelconque des revendications 12 à 14, dans lequel l'éprouvette (3) comprend une mousse, un non-tissé, un tissu, un tricot et/ou un film.

16. Procédé selon l'une quelconque des revendications 12 à 15, dans lequel le milieu de liaison (6) comprend un fluide corporel animal ou humain.

17. Procédé selon la revendication 16, dans lequel le milieu de liaison (6) comprend du plasma sanguin coagulé avec une solution de calcium.

18. Procédé selon l'une quelconque des revendications 12 à 15, dans lequel le milieu de liaison (6) comprend un liquide qui contient un constituant présent dans un fluide corporel animal ou humain.

19. Procédé selon l'une quelconque des revendications 12 à 18, dans lequel l'éprouvette (3) est pelée du substrat (4) à un angle de 0° à 180°, par rapport à la position de l'éprouvette (3) dans le creux (5) du substrat (4) avant le pelage.
